(19)

Europäisches
Patentamt
European
Patent Office
Office européen
des brevets

(11) **EP 4 176 926 A1**

(12) **EUROPEAN PATENT APPLICATION**

(43) Date of publication:
**10.05.2023 Bulletin 2023/19**

(21) Application number: **21207247.4**

(22) Date of filing: **09.11.2021**

(51) International Patent Classification (IPC):
**A61N 5/10** *(2006.01)*  **G16H 20/40** *(2018.01)*
**G06N 3/08** *(2023.01)*  **G06N 20/00** *(2019.01)*
**G16B 40/00** *(2019.01)*

(52) Cooperative Patent Classification (CPC):
**A61N 5/103; G06N 3/08; G06N 20/00; G16H 20/40;**
**G16H 30/20; G16H 30/40; G16H 40/63;**
**G16H 50/20; G16H 50/70;** A61N 2005/1041;
A61N 2005/1074

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **BONDAR, Maria Luiza**
**Eindhoven (NL)**

• **FLAESCHNER, Nick**
**Eindhoven (NL)**
• **WEESE, Rolf Jürgen**
**Eindhoven (NL)**
• **NEUKIRCHEN, Christoph**
**Eindhoven (NL)**
• **HEESE, Harald Sepp**
**Eindhoven (NL)**
• **WIEGERT, Jens**
**Eindhoven (NL)**

(74) Representative: **Philips Intellectual Property &**
**Standards**
**High Tech Campus 52**
**5656 AG Eindhoven (NL)**

(54) **A MACHINE LEARNING SYSTEM FOR GENERATING MULTIPLE DOSE DISTRIBUTIONS CONDITIONED BY DIFFERENT PLANNING TEMPLATES OF CLINICAL GOALS**

(57)  A computing system (SYS) and relates method for supporting radiation therapy planning. An input interface (IF1; UI-I) is provided for receiving input radiation treatment plan templates, or types of such plan templates, for plural radiation treatment plans. A trained machine learning model (M) of the system (SYS) is configured to compute plural dose maps associated with the received radiation treatment plan templates or with the types of such plan templates.

**FIG. 1**

**Description**

FIELD OF THE INVENTION

The invention relates to a system for supporting radiation therapy planning, to a training system for training a machine learning model for use in such a system, to a radiation treatment arrangement, to related methods, to a computer program element, and to a computer readable medium.

BACKGROUND OF THE INVENTION

Cancer is a leading cause of death in industrialized nations around the world. Well over half of cancer patients receive treatment by Radiotherapy ("RT"). In RT, high energy ionizing radiation is applied to the patient, either from outside the patient (external RT) or from within (brachytherapy). The goal is to destroy cancerous tissue whilst preserving non-cancerous, healthy tissue. In some types of external RT, X-radiation in the megavolt (MV) range is used. The total radiation dose is administered in portions (also known as "fractions") over time as per a treatment plan, such as once per day over the course of 2-4 weeks for example.

The preparation of the treatment plan is an important but time-consuming process. The user of a Treatment Planning System often strives to come to favorable compromise between tumor irradiation and sparing of normal tissue.

A radiation therapy plan can be generated using machine learning ("ML")- models trained to predicted dose distributions, if desired in 3D. For example, for a given patient, dose distribution may be predicted using a trained artificial neural network, a CT image, contours of the target (PTV) and organs-at-risk (OAR). However, such approaches are always based on a single "best" dose distribution.

In the past, Multicriteria optimization (MCO) was proposed as an optimization method that can produce multiple dose distributions which the user can examine. An aim in MCO is to approximate a Pareto set for the multi-objective optimization problem with conflicting objectives. Solving an MCO problem results in multiple solutions on the Pareto set.

Different solutions along a Pareto front of the Pareto set can be obtained by minimizing a weighted sum for different settings of the weights (e.g., different combination of values). However, this is a cumbersome, time-consuming undertaking with sometimes uncertain results, as not all solutions along the Pareto fronts are clinically acceptable solutions. Also, the dimensionality of the Pareto front is determined by the number of objectives, meaning that the user might need to explore a very large set of Pareto solutions to find a clinically acceptable solution. In summary, MCO can result in a large set of clinically unacceptable solutions for a fixed choice of planning goals.

Whilst machine learning approaches exist, such approaches merely produce a single "best" dose distribution. Even when ML generate, existing dose distributions as may be held in a medical database may be similar to a certain average of previous plans in the database, and may hence fit not well to an out-of-average patient. In addition, existing plans in a medical database may have been optimized with various different planning goals in mind. But the individual planning goals of each plan in a plan database may be unknown to the user. The existing dose distributions in the database might not satisfy a user selected clinical plan template of planning goals. Consequently, the user might not approve the use of AI-dose distribution for planning. In previous approaches, the user could not choose between different dose distributions corresponding to different sets of plan templates of clinical goals (e.g., variations of clinical dose limits to organs at risks, or various trade-offs between target coverage and organ-at-risk sparing). However, generating multiple plans from scratch for each patient either by manual or automated planning is not feasible busy clinical environments.

SUMMARY OF THE INVENTION

There may therefore be a need for improved treatment planning.

An objective of the present invention is achieved by the subject matter of the independent claims, wherein further embodiments are incorporated in the dependent claims. It should be noted that the following described aspect of the invention equally applies to the training system for training a machine learning model, to the radiation treatment arrangement, to the related methods, to the computer program element, and to the computer readable medium.

According to a first aspect of the invention there is provided a computing system for supporting radiation therapy planning, comprising:

> an input interface for receiving input radiation treatment plan templates, or types of such plan templates, for plural radiation treatment plans, and
> a trained machine learning model configured to compute plural dose maps associated with the received radiation treatment plan templates or the types of such plan templates.

In embodiments the input interface is arranged as a user interface, configured to allow a user to select the input radiation treatment plan template or type.

In embodiments the input interface is configured to allow selection of any one or more of the input templates and the plural dose maps are computed by the trained machine learning model in response to such selection.

In embodiments the system further comprises an output navigation user interface, configured to allow a user to navigate through the output plural dose maps and select a dose map from among the plural dose maps.

In embodiments the output navigation user interface and/or the input interface is arranged as a graphical user interface, GUI.

In embodiments the trained machine learning model is of the generative type.

In embodiment, the templates include data that relates to different clinical goals.

Such as template may relate or represents a "clinical goal" in respect of a given patient. For example, one example of a clinical goal may be formulated as target coverage and high sparing of organs A, B, and lower priority sparing of organ C. Another clinical goal 2 envisages target coverage and low priority sparing of A, and high priority sparing of B and C. Yet another clinical goal may relate to a time objective for delivery of the treatment, such as for example delivery time under *2 minutes* and acceptable target coverage. A clinical goal is described via a set of objectives as per the template. The type and number of objectives can be different between different "clinical goals", but may be related for templates of the same type, for example, with numerical differences in terms of dose limits and/or priorities but with similar effect.

In embodiments, the said clinical goals includes any one or more of: i) different clinical dose upper limits for one or more organs at risks, ii) different dose lower limits for a target, iii) different trade-offs between target coverage and organ-at-risk sparing iv) different treatment parameters. Examples of treatment parameters may include treatment delivery time, monitor units, treatment modality (photon, proton).

In embodiments, the templates are encoded as entries of vector or matrix. Surrogate data correlated or representable of such templates or their types may be used instead, such as specification of side effects to be avoided, or desirable effects etc, imagery etc.

In embodiments, the system may include a radiation therapy planning module configured to generate a treatment plan based on the dose map selected by the user via the output navigation interface.

In another aspect there is provided a training system configured to train a machine learning model based on training data to obtain the trained model of the system.

In another aspect there is provided a radiation treatment arrangement, including a planning system, a system as defined above in any one of the above embodiments, and/or a treatment delivery device.

In another aspect there is provided a computer-implemented method, comprising:

receiving input radiation treatment plan templates, or types of such plan templates, for plural radiation treatment plans, and

computing by a trained machine learning model plural dose maps associated with the received radiation treatment plan templates or the types of such plan templates.

Method for training a machine learning model based on training data to obtain the trained model in the system of any one of the above-mentioned embodiments or aspects. training may include adapting parameters of the model. Training may implicit or explicit. Explicit modelling includes artificial neural network models, regression models. etc. Implicit modelling may include automatic, semiautomatic or manual clustering or training data, such as a database of plan templates. The plan templates may be clustered into types for example using a clustering algorithm for example. Pre-processing templates into types using manual grouping are also envisaged in combination with explicit modelling. A pre-preprocessing machine learning model may be used to map. in deployment, new plan template on a class/label/type/category/latent variable, etc. It is the so mapped entity that is then processed by the model to predict the associated dose distribution.

In another aspect there is provided a computer program element, which, when being executed by at least one processing unit, is adapted to cause the at least one processing unit to perform any one of the methods.

In another aspect there is provided at least one computer readable medium having stored thereon the program element or having stored thereon the trained machine learning model.

Whilst in the past some form of ML approaches to generate a dose distribution for calculating an RT plan may have been proposed, such proposals always focus on producing the "best" single dose distribution. However, what is proposed herein is to use a set of different planning templates as part of the input in the computing system, and have the system generate a set of dose distributions to be presented to the user as alternative options. Thus, the proposed system allows prediction of multiple dose distributions fulfilling different planning goals as represented by the plan templates. Different dose distributions may be explored to find a suitable one and hence its associated plan template for the subsequent planning.

Preferably, for better robustness and better variability, the ML model operates on plan template types as opposed to individual plan templates.

By using an ML approach in the proposed setup, no handcrafted features are required which reduces time and increases throughput.

In the proposed approach, training data is based on plan templates fulfilling different planning goals as may be represented either individually or by the plan template type for example. Thus, as envisaged herein, the ML model is preferably trained on different, multiple planning templates/types representing different clinical goals. The plan templates or types may be formulated explicitly or implicitly via correlated surrogates, such as side effect specifications etc. In the following, a reference to a plan template is understood to also refer to a plan template type.

The proposed system envisages ML-based prediction of multiple dose distributions and may facilitate navigation between multiple plan templates, based for example on a system of supported user interface, preferably of the GUI type.

In sum, what is proposed herein in embodiments is using the trained ML model to predict, before plan optimization, *n* different dose distributions corresponding, respectively, to *n* different plan templates /types. The different plan templates (or planning "flavors") may correspond to e.g., different priorities for sparing of organs at risk ("OAR") for the same set of target objectives, or to similar priorities in sparing organs at risk, but for a different set of target goal objectives. The type of the plan template or the plan template may be encoded in a vector, matrix or tensor of latent variables. The user may navigate or explore a range of actual or potential, predicted set of dose distributions, for example by optionally adjusting value(s) of the latent variables.

For the proposed method, clinically accepted plan templates can be created in the training data set. Various options are envisaged herein: a manual process, semi-automated as selected from the results of one or more MCO runs or other planning techniques with different planning templates, semi-automated using different planning templates for example. The training data can be extracted from a historical database of clinically accepted plans of previously treated patients.

*"user"* relates to a person, such as medical personnel or other, operating the RT delivery apparatus, or overseeing or taking part in the treatment or delivery procedure. In other words, the user is in general not the patient.

In general, the term *"machine learning"* includes a computerized arrangement (or module) that implements a machine learning ("ML") algorithm. Some such ML algorithms operate to adjust a machine learning model that is configured to perform ("learn") a task. Other ML operate directly on training data, not necessarily using such as model. This adjusting or updating of training data corpus is called "training". In general task performance by the ML module may improve measurably, with training experience. Training experience may include suitable training data and exposure of the model to such data. Task performance may improve the better the data represents the task to be learned. *"Training experience helps improve performance if the training data well represents a distribution of examples over which the final system performance is measured'.* The performance may be measured by objective tests based on output produced by the module in response to feeding the module with test data. The performance may be defined in terms of a certain error rate to be achieved for the given test data. See for example, T. M. Mitchell, "Machine Learning", page 2, section 1.1, page 6 1.2.1, McGraw-Hill, 1997.

*"[RT] Plan template"* or *"[RT] planning template"* is used herein synonymously and includes data that may be processed by a planning algorithm to obtain a plan. Plan template is data available pre-planning. Plan template may represent an individual clinical goal or objective. *"[RT] Plan"* incudes the result of planning algorithm processing the plan template. The plan includes in particular control parameters for controlling operation of an RT treatment device, such as a linac.

*"[RT] Plan template type"* or *"[RT] planning template type"* includes classes of templates obtained by categorizing, clustering plan templates according to some criteria, such as the manner of sparing which organs at which priority, or other general clinical goal or objective. Thus, plan template types unify individual goals as per the induvial plan template.

## BRIEF DESCRIPTION OF THE DRAWINGS

Exemplary embodiments of the invention will now be described with reference to the following drawings, which, unless stated otherwise, are not to scale, wherein:

Fig. 1 shows a block diagram of an arrangement for radiation therapy;
Fig. 2 shows a block diagram of a system for supporting radiation therapy planning;
Fig. 3 illustrates operation of a machine learning model configured to predict dose distributions based on radiation treatment plan templates;
Fig. 4 is an illustration of input data that may be used by a machine learning model for predicting dose distributions;
Fig. 5 shows a schematic visualization of a elements of a graphical user interface according to one embodiment as may be used in the system of Fig. 2;
Fig. 6 shows a schematic visualization of elements of a graphical user interface according to another embodiment;
Fig. 7 shows a schematic diagram of a machine learning model;

Fig. 8 shows a block diagram of a training system for training a machine learning model;

Fig. 9 shows a flow chart of a computer implemented method for supporting radiation therapy planning; and

Fig. 10 shows a flow chart of a method of training a machine learning model.

## DETAILED DESCRIPTION OF EMBODIMENTS

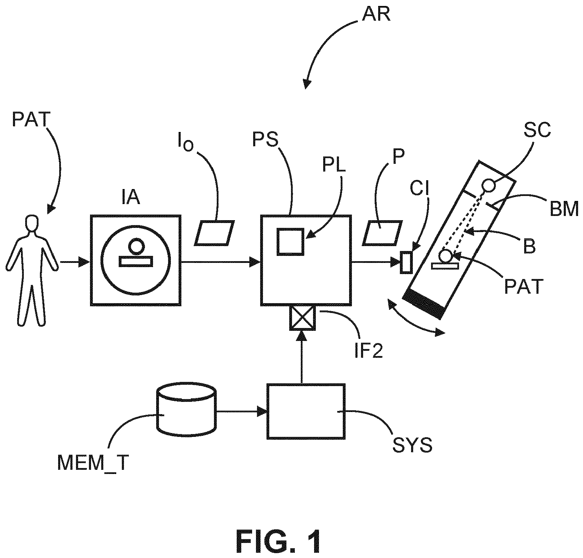

With reference to Fig. 1, there is shown a schematic block diagram of a computerized radiation therapy ("RT")-arrangement AR. Broadly, the RT arrangement AR may include a treatment device TD configured for RT delivery based on a treatment plan P. The arrangement AR may further include a treatment planning system PS configured to compute the said plan. Very broadly, as proposed herein, the arrangement further includes a system SYS configured to assist a user in exploring dose distributions based on planning templates, in order to find a suitable such planning template that fits the needs for a current given patient. It is the so identified planning template that may then be used by planning system PS to compute the plan P for treatment. Before explaining operation of the new arrangement in more detail, some context around RT is provided first.

External radiation therapy is mainly envisaged herein although the principles described herein may also be applied to internal radiation therapy. In external radiation therapy, high energy radiation is delivered to a patient to kill off cancerous tissue. The objective is to kill off as much of the cancerous tissue as possible, whilst sparing non-cancerous tissue. The cancer killing radiation is delivered by the treatment device TD such as a linear accelerator ("linac"). In linacs, a beam B of high energy x-ray photons, such as in the megavolt range are generated by a source SC. During treatment, the patient is exposed in a pre-mediated manner to the beam and thus the radiation delivered by the treatment device TD.

Types of X-ray based external RT envisaged herein include intensity-modulated radiation therapy ("IMRT"), volumetric-modulated arc therapy and others. Specifically, in X-ray based external RT, the radiation is delivered at certain doses from a multitude of different spatial directions. More specifically, radiation treatment beams propagating along the said different spatial directions can be made to intersect preferably at the lesion (the target volume, "PTV") to be treated, thus delivering a higher dose there, whilst leaving a lower dose at surrounding non-cancerous tissue, in particular organs at risk ("OAR"). The applicable doses to be delivered and the spatial directions relative to the lesion site through which the dosages are to be delivered are defined in the treatment plan.

The treatment plan is computed by a planning module PL of the treatment planning system PS. The planning module PL may be implemented by a computing system.

Computing the treatment plan is in general a complex mathematical operation that may consume time and computational resources, and hence electrical energy. Preferably, high performance computing processors such as those of multi-core design may be used to secure a reasonable return time.

Computing the treatment plan ("the planning problem"), may be formulated as an optimization the problem. Specifically, one or more objective functions are formulated subject to one or more constraints. Generally, the objective function and the constraints define the manner in which the treatment dose is to be delivered given location, shape and extent of the cancerous lesion. More specifically yet, the objective function(s) and the constraint(s) describe how much dosage is to be delivered in which regions. A clinician prescribes for instance the average dose in a certain area, or the maximum or minimum dose to be delivered and formulates these objectives and constraints.

Planning imagery is previously acquired by a medical image modality IA, such as a computed tomography scanner CT, a magnetic resonance imager (MRI), or a nuclear imaging modality such as PET/SPECT. Based on this planning imagery $I^0$, the cancerous target region/volume PTV may be identified. This initial planning imagery $I^0$ is preferably 3D so as to capture the 3D shape and extent of the cancer lesion situated usually at least partly, if not fully, inside the patient. The target volume TV is identified either manually by delineation in the imagery, or the delineation is done automatically or semi-automatically by a segmentation algorithm. The identified tissue includes the volume PTV to be treated where the cancerous tissue resides plus a certain safety margin. Volumes of non-cancerous tissue, such as the above-mentioned OARs, may also be segmented or otherwise identified in the planning imagery. OARs are to be spared from radiation exposure, or deposited radiation is at least not to exceed certain maximum thresholds as formulated in the planning constraints.

The planning module PL implements the said planning algorithm to find a solution to the planning problem. In particular, inverse planning algorithms may be iterative and compute how much dosage is to be delivered from which spatial position over a certain period of time. In the iterations, the objective function is improved until an acceptable treatment plan is found. The total dose is deliverable in fractions. In other words, parts of the dose are delivered over a treatment course of days or weeks, in multiple treatment sessions. A treatment session for a fraction is usually once per day. In each such treatment, a certain dose part of the total dose envisaged is delivered from the computed spatial positions.

The computed RT treatment plan P may include in particular a quantity known as a "fluence map". The fluence map $\varphi$ defines the intensity of the treatment beam across its cross section.

Based on the computed fluence map, a machine control program is computed by the planning module PL to control operation of the treatment delivery device TD (such as the linac or other). For example, in IMRT, a leaf sequencing tool computes the control program to control a beam modulator BM of the delivery device TD. For example, the beam modulator may include a multi-leaf collimator. The control program may control position and timing motions of leaves of the MLF collimator. For instance, in modulated radiation therapy, the treatment device TD includes a treatment head with the beam modulator BM such as the said multi-leaf collimator that can realize different beam shapes. The cross section of the beam B may be divided into beamlets where different dosages can be realized in a spatially resolved manner. The computed control program of the treatment plan may prescribe different collimator settings that may vary as the treatment head is moved (by a rotatable gantry) to different spatial positions around the target volume. The collimator settings may prescribe how leaf elements of the multi-leaf collimator are to be moved to realize the different beam shapes. The control parameters may be passed via a control interface CI to the delivery apparatus TD to effect the treatment.

Preferably prior to actual plan delivery/treatment, as a quality check for example, a simulation tool of the planning system PS may process the control program to compute deliverable dose distribution $d$, if the control program (that is, the current plan) were to be used in treatment. The computed dose distribution $d$ may be stored as a spatially resolved file or data structure, also referred to herein as the dose map. The deliverable dose distribution map $d$ may be optionally superimposable on the planning image to represent the dose per location. The dose maps d envisaged herein are preferably 3D, although formulations in 2D are not excluded herein.

In order to further facilitate RT planning, the arrangement may include the above-mentioned dose explorer (system) SYS. The dose explorer SYS may be arranged as a stand-alone system so is not necessarily integrated in an RT arrangement AR (in particular in the planning system PS) as shown in Fig. 1. Both options are envisaged herein.

Broadly, the system SYS operates as a dose explorer that allows a user to explore various planned dose distributions, given different RT plan templates as input. As will be explained in more detail below, each plan template includes information that may be submitted to the planning system PS/planner module PL to compute a usable plan $P$ for controlling the delivery apparatus TD during actual RT. But before committing such a plan template to the planning system PS, and thus binding time and computational resources, the user can explore before various predictions of dose distribution maps (referred to herein simply as "dose" or "dose map") that may be obtained if a respective plan template were to be used for planning. In addition, the system SYS allows addressing the above-described deficiency in particular with machine learning (ML) based dose prediction solutions. Such ML systems were trained before on training data including previously computed plans. Training data may however be biased, with limited variability and this bias then tends to feed through to the model trained on such data. The training does not represent well the underlying distribution of plans. As one example, such bias may for example emerge in certain geographical area where patients for some reason tend to present with similar cancer conditions. This may call on the medical facility that serves this area to draw up similar clinical plans with similar characteristics. But such plans may not fit well the plan requirements for a patient that presents with a markedly different cancer condition for example. An ML system trained on such data may thus not well predict the expected dose. Whilst data augmentation techniques can be used to somehow alleviate a lack of variability in training data, this is expensive to do and does not always lead to models that learn to generalize well. The above may also lead to the undesirable situation of inconsistent treatment. The same patient may receive different treatments at two different clinics because one clinician might prefer sparing OAR 1, whilst another clinician may feel that OAR 2 deserves better sparing, etc.

So as to address these shortcomings, what is proposed herein is the dose explorer system SYS including a machine learning model M trained at the outset on a range of plan templates or plan templates of different type to ensure variability in dose prediction. In addition (or instead), the dose explorer system SYS supports "dual" user interaction in respect of input and output by a sub-system of user interfaces UI. The interfaces UI may be arranged as graphical user interface(s) ("GUI"). The system SYS controls the interfaces to allow user to interactively explore a larger range of possible dose distributions, as a function of a range of possible planning templates. In some embodiments, the system is configured to take in as input multiple plan templates, and computes, based thereon, corresponding predicted dose distributions for the templates, one or more such dose distribution maps per input plan template or type. The system allows the user exploring (such as scrolling through) already available or newly created plan templates with different characteristic ("flavors" as it were), and observing how the associated dose distributions vary with different such plan templates. The dose explorer thus allows user to find the correct dose template for their needs, that is the one that is likely to give rise (after planning) to a dose distribution that fulfills the requirements for a given patient at hand.

The proposed explorer system may thus comprise the trained machine learning model M, trained on suitably varied training input data that reflects different planning templates of different type. A type or "flavor" of a planning template may relate to the manner in which a given OAR is spared for example. A template type may spare some OAR(s) more than other(s). Sparing may be by specifying lower dose level for an OAR and/or by awarding different priority weights of different OARs. The trained model is controllable by the user interface UI to produce, based on multiple templates, respective multiple dose distributions. The (sub-)system of computer supported user interfaces UI in communication with the model M are configured with functionalities that allow user to examine, navigate or select the input templates and/or the associated, predicted, output doses. The input and/or output may be displayed on a display device, preferably upon user instructions as received by a graphics display generator through the system of user interfaces.

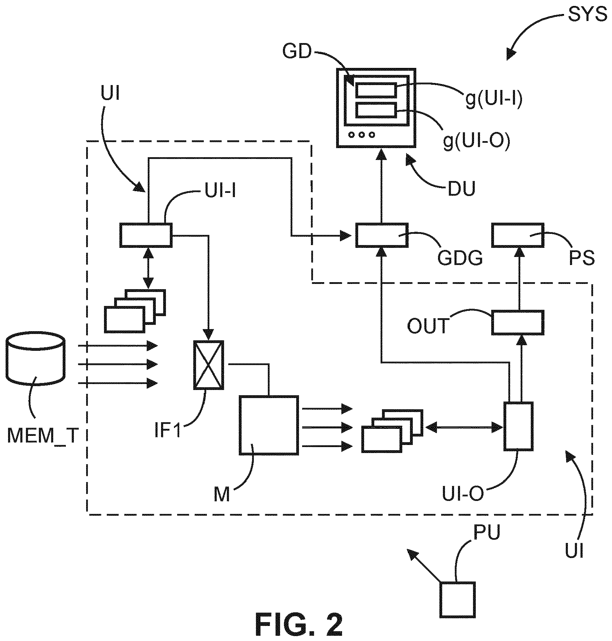

Fig. 2 shows a schematic block diagram of components of the computer implemented dose explorer SYS. The user interface (sub-)system UI supported by system SYS includes an input data user interface UI-I facilitating inputting data such as template plans into the system SYS. The input data user interface UI-I, or input explorer interface, allows user to identify/retrieve, manipulate, create or view plural plan templates. Extant plan templates from previous or the same (current patient) may be held in a memory, storage, etc, such as a database MEM_T. The input explorer user interface UI-I allows user to supply one or more such plan templates Ti to input interface IF1 for processing by trained machine learning model M. ML model M processes the templates Ti in sequence or at once to produce corresponding set of predicted dose distributions, one or more dose distributions per respective input planning template as identified by input user interface UI-I.

The input plan templates may be displayed on display unit DU in a graphics display GD generated by a graphics display generator GDG.

The graphics display GD may include graphical user interface elements g(UI-I) of the input interface UI-I.

The output data (the predicted dose distributions) may be explored by an output data user interface UI-O, also referred to herein as "output explorer interface". The generated graphics display GD may include graphical user interface elements g(UI-O) for the output data user interface UI-O. The two GUI representations g(UI-O), g(UI-I) may include interactive widgets, display panels, etc. which may be combined into a single graphics display for concurrent display as schematically shown in Fig. 2, but this is not a necessity herein and separate renditions in different windows, or indeed on different display devices, are also envisaged herein. However, the combined rendition is still preferred herein as this allows better user guidance during interaction and the preferred option to display the dose vs template variation for better in-depth exploration.

The input templates and/or the output associated dose distributions may be displayed in sequence or at once concurrently. In particular, each dose distribution $D^{Ti}$ and its associated plan template $T_j$ may be displayed concurrently as a respective associated pair $(T_j, D^{Ti})$. The output user interface UI-I and/or input user interface UI-O are preferably operable in co-operation to allow user scrolling through both, the input templates and output dose maps. In this manner, the user can identify the "best" template that causes a dose distribution that best fits the task at hand. Once the best dose distribution and its associated template, the best template vs best dose pair $(T_j, D^{Ti})$ is identified, the plan Ti can be passed on to the planning system PS via output interface UI-O. To this end, the graphical rendition g(UI-O) of output interface UI-I may include a "commit" or "plan" button or similar element that causes interfacing with the planner PL via interface IF2 and data transfer to planner PL. The planner PL may commence the planning operation by executing the plan optimization algorithm to compute the plan P, based on the template $T_j$ and/or the associated dose $D^{Ti}$. The planner PL essentially "translates" via the planning optimization algorithm the selected template $T_j$ into fluence maps and/or delivery control parameters for the delivery device TD. The system SYS thus facilitates saving time and computational resources by committing only promising templates. This avoids putting in speculative planning queries for some plan templates that ultimately (post-planning by planner PL) may not result in acceptable dose distributions, a practice likely to be prohibitive, in particular in ever busy clinical settings.

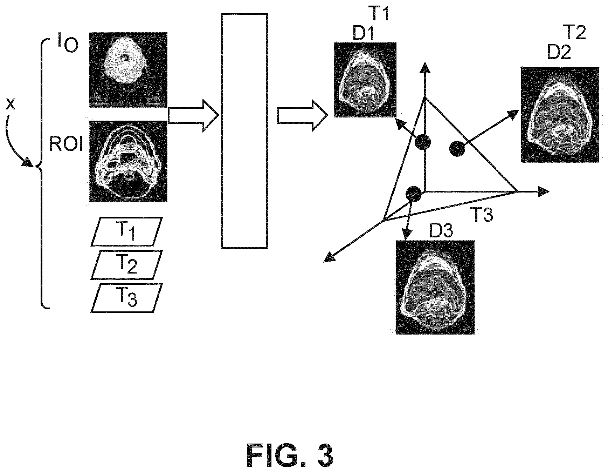

Fig. 3 illustrates operation of system SYS. Input data x is supplied to the trained machine learning model M through the input interface UI-I. The input data includes one, but preferably more than one, planning templates. Some, or each, such input data x may include plan template Tj which may include definitions of delineated/segmented regions of interest including OAR's and PTV's, and prescribed dose expectations, preferably formulated in terms of dose limits, in particular minimum and maximum dose for PTV and OARs, respectively. Optionally, image data is included instead or in addition to the foregoing, in particular the planning image 10 and imagery of the delineated/segmented OAR's and PTV's. The imagery of the delineated/segmented OAR's and PTV's may be part of the input planning image $I_0$. The delineated/segmented OAR's and PTV's may be designated by an associated segmentation map for example, associated with the input image.

Plural such templates Ti are processed by the machine learning model M into respectively associated predicted dose distributions $D_i^{Ti}$. Three such input templates T1-T3 are illustrated, along with their respective predicted dose distribution $D_1^{T1} - D_3^{T3}$, three in number, one for each template. Indicators for the dose distributions $D_i^{Ti}$ may be rendered as points in an $N$-dimensional space, with N representing the number of templates $T_i$ processed by the machine learning model, and hence the number of predicted doses $D_i^{Ti}$.

For example, in the illustration of Fig. 3, $N = 3$ and the graphics display GD includes a rendition of a three-dimensional portion of Euclidean space with three axes. The predicted doses $D_i^{Ti}$ associated with the templates are rendered as points $p_i$ on a polyhedral surface SF. Renditions of such indicators (points $p_i$, crosses, or other apt symbology) on a surface SF as shown in Fig. 3 is optional, but may be beneficial for better comparison of quality metrics in relation to the templates/dose maps. The axes may relate to plan template type. As an alternative or instead, the axes may relate to a certain quality measure(s) of the associated dose. The points-on-surface rendition may thus facilitate visualization of proximity of the dose maps with varying templates in respect of the quality measure. This allows probing dose behavior and how this varies with respect to the quality measure. The user may request through the user interface UI-I, UI-O cycling through different such quality measures which causes a dynamic update of the point-on-surface visualization, with the N points, in an apparent "motion", forming or assuming different mutual spatial configurations.

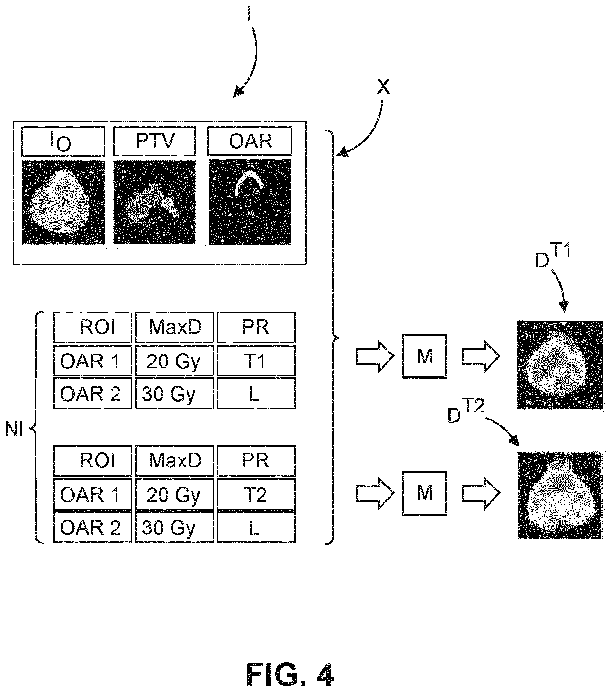

Thus, in the example of Fig. 4 the three axes correspond to three dose quality measures, and three dose distributions correspond to three templates. User interface UI can support comparison of doses corresponding to any number of templates.

For example, one axis may relate to a total plan quality metric score, whilst another axis relates to a plan quality score for targets, whilst the third axis relates to a plan quality score for OARs. Other quality metrics can be associated to the axes, e.g, target homogeneity score, delivery time, etc. A plan quality metric score is described by B. E. Nelms et al, in "Variation in external beam treatment plan quality: An inter-institutional study of planners and planning systems", published in Pract. Radiat. Oncol., vol. 2, no. 4, pp. 296-305, (2012).

Some or each of indicator(s) $p_i$ (with on-surface visualization or not) as rendered may be interactive and can be selected by a pointer tool or by touch screen action to cause a selection event. Such a selection event may elicit a display operation to effect visualizing the associated dose distribution $D_i^{Ti}$ associated with template $T_i$. The dose map may be illustrated as shown by superposition on a medical patient image, such as on the planning image 10. Again, the system of indicators as points-on-surface or other, is optional. In some simpler embodiments, upon detection of a user selection event by input interface, the computed dose maps $D_i^{Ti}$ are displayed in a graphics display, either all concurrently which is preferred, or concurrently but per a user defined sub-group of dose maps rather than all such dose maps, or in sequence, either one-by-one, or again group-by-group, etc. Other visualization protocols are also envisaged.

It will be understood that the explorer system SYS is dynamic and preferably with (quasi-) real time operation. Specifically, the graphics display GDG is configured to update the displayed dose maps as a function of selected templates Ti. The displaying of the dose maps is dynamically updated as the user feeds new templates Ti or (new) combinations of templates Ti into the machine learning model M. This dynamic adaptation of the graphics display GD is also envisaged in embodiments where dose maps indictors $pi$ are used, such as the points-on-surface as illustrated in Fig. 3: the dose distribution indicators are in generally repositioned as the template input is adapted. This dynamic display adaption in response to template input variation affords in embodiments an on-going, quasi-continuous user support during the navigation/exploration of the template-versus-dose distribution relationship.

Whilst in Figs. 2,3 a single machine learning model is shown that processes all or some of the templates in sequence or at once, such an embodiments, although envisaged herein, is not at the exclusion of alternative embodiments. Such alternative embodiments may envisage a bank of separately trained machine learning models, one or more for each template type for example.

Turning now to Fig. 4, this shows a further illustration of operation of dose explorer system SYS, in particular with more details on the nature of the input data processable by the (one or more) machine learning model M to produce the associated dose maps $D_i^{Ti}$. The input data x may be made up of image data $I$ and non-image NI. The non-image data T includes in particular the plan template data that describes requirements for the dose distribution in terms of dose limits and, optionally, their priorities. The image data I is spatial data such as 3D image data mainly envisaged herein. It may include the earlier mentioned planning image 10, and/or associated segmentations for the PTV(s) and/the OAR(s).

The planning image $I_0$ may be a slice of a 3D image such as a CT scan, MRI or PET or other, the planning image is a 3D volume. The regions of interest, such as the PTV or OAR, can be represented as 3D segmentations, for example by using binary maps etc. The non-image NI template data may be organized in respective matrices, eg, one matrix per template as shown in the lower left of Fig. 4. The matrices encode respective associations of regions of interest with their respective dosage requirements (min-/max-limits). Specially, a template Ti matrix may include rows and columns, indicative of the respective regions of interest (such as OAR and/or PTV) versus the associated maximal or minimal dosage requirements. Optionally, for each dose requirement, there is a priority weight PR. The weights, like other elements of the template, form instructions for the panning system PS. Such priority weights allow defining plans with different OARs sparing modes or "flavors"/types as briefly mentioned earlier. For example, as illustrated in Fig. 4, the maximum allowable dose distributions for organs at risk OAR1,2 are the same for templates T1 and T2. However, the plan templates differ in their respective priority weights that are attached to those limits. For example, plan template T1 tends to spare OAR1 more than does template T2. Template T2 spares OAR2 more at the expense of OAR1. There may be similar priorities when multiple PTVs are treated, in that minimum dose limits are associated with respective priority weights. A plan that spares, say OA1 as does T1, but uses a different numerical dose limit may thus be still of the same template type as T1 for example. Definition of template types however may differ from application to application and may be defined differently across facilities.

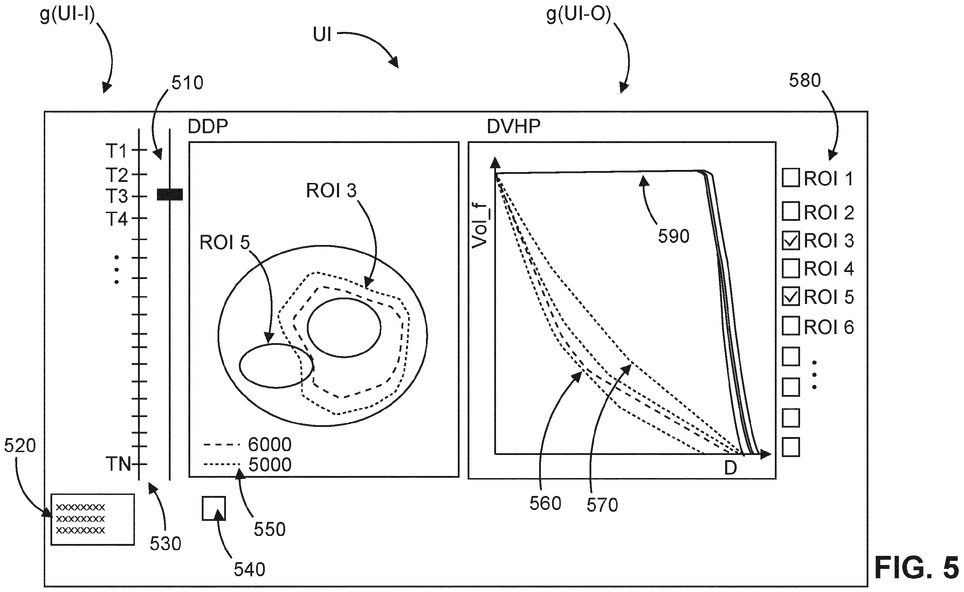

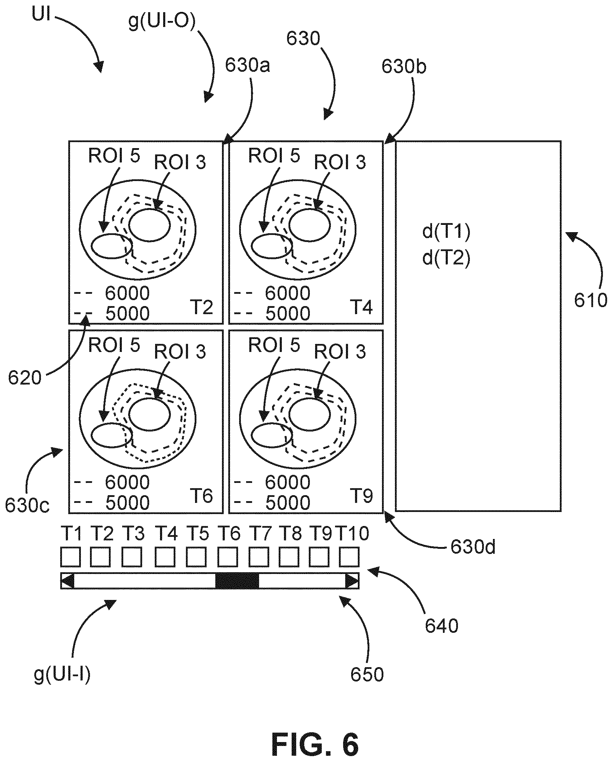

Turning now to Figs. 5,6, input and output explorer user interfaces UI-I, UIO are schematically illustrated. The Figures illustrate embodiments of graphics displays for a GUI where input and output explorer interfaces UI-I,UI-O are combined although this may not necessarily be so in all embodiments. However, such a combined rendition may be preferred herein for user support efficiency as mentioned above. Generally, the GUI includes elements to support the user in selecting, manipulating, navigating or reviewing input data x and the output (the associated dose maps) y= $D^x$.

There are preferably also elements such as viewing panels where the input and/output data are displayed. Although not illustrated explicitly, there may also be a dispatch GUI element that allows user to pass on a selected template, optionally along with its associated dose map, to the planner PL to initiate executing the planning operation by planning algorithm.

In general, various GUI elements (or widgets) are described below in connection with Figs 5,6, each representing a functionality. Some widgets are interactive. Such interactive widgets may include an element that accepts user input. The interactive widget is associated with a respective event handler. The event handler causes the associated functionality to be executed by an underlying computing system PU. The computing system PU may include components such as one or more processors, memory, etc that are operable to implement the functionality.

Turning now first to Fig. 5, this shows a graphical interface GUI according to one embodiment for input data and/or output data exploration, preferably for both.

Broadly, the graphics display for the GUI includes elements for the graphical rendition g(UI-I) of input interface UI-I and/or elements for the graphical rendition g(UI-O) of output interface UI-O.

The input interface elements envisaged herein allow in particular selection of plan templates Ti from a database for example. Specifically, said elements may include selection elements 510, 530 for the user to select one, but preferably two or more templates. The selection elements 510, 530 may include, as shown, a user operable/interactive slider element 510, optionally arranged alongside a graduation bar 530. Graduation bar 530 indicates via respective identifiers "T2, T2, T3" etc, the respective template selectable by slide 510. For example, a slider element may be dragged by user interaction along slider bar 510 and the user can request, by pointer action (mouse-click, touchscreen action, etc), selection events for templates based on the identifiers as the slider is dragged along the graduation bar 530. Any other selection elements configured to allow user to select one, but preferably plural templates, are also envisaged herein. The selection means 510, 530 are configured to allow for selection of templates in sequence for example. However, multi-selection options where multiple templates are selected at once are also envisaged, such as with a tick box bar element or similar.

Upon selection, a memo-text widget 520 may optionally display a textual description of the respective template. In other words, text info box 520 is updated as slider 510 is operated by the user. The textual description may include a short mnemonic description such a "OAR2 sparing". The textual or other mnemonics may be assigned by user during selection for later reference. The textual or other mnemonic may relates to the template type or may relate to the induvial template, as required.

An optional element 540 allows selection of ROI goals and priorities for the selected template. Specifically, upon user activation, a window display widget may launch to indicate ROI optimization goals and/or their priorities as per the currently selected template(s). In case of multi-template selection, the window of text widget 520 includes such information for each template. The information in template-info window may include respective matrix type representation for dose limits and/or their priorities as explained above in connection with Fig. 4. The template data may be dynamically modified, changed, updated which causes a dynamic re-computation by model M of dose $y$ and optionally an update of a dose display panel.

The graphic display generator also includes one or more such display panels DDP, DVHP to display a visualization of the dose distribution(s) computed by model M for each selected template $T_i$. Figs. 5 illustrates two panels DDP DVH. Whilst this is indeed envisaged in embodiments, this is not a necessity herein and the GUI may include instead merely one such panel, either panel DDP or panel DVHP. For example, dose map(s) may be displayed in panel DDP to visualize spatial distribution of the predicted dose, preferably relative to the planning or other medical patient image. For example, the dose distribution may be shown in isoline presentation as shown in panel DDP, with dose distribution optionally superimposed on an image, such as planning image 10. The image may represent region(s) of interest (eg, PTV or OARs) to which the isolines pertain. The regions of interest, eg ROI5, ROI3, may be indicated as segmentations. As shown, isolines may be rendered in visualization panel DDP as closed curves in solid, dashed or other line style. Optionally, a legend element 550 is displayed alongside, indicative of the respective dose levels.

In addition or instead of such isoline visualization of dose distributions, a histogram representation may be visualized as shown in panel DVHP.

As mentioned earlier, the interactive and dynamic nature of graphics display GD is cause by the graphics display generator updating the visualizations in DDP, DVHP as the user selects the templates with selection means 510, 530.

Optionally, as part of the output explorer interface UI-O, its graphical rendition g(UI-0) may include similar selector element 580 as does graphical element g(UI-I) to allow user selecting individual's region of interest ROI, for example, OAR's or PTV's, for, or in, the respective dose distribution. This ROI selection element 580 can be implemented as a slider bar element (with or without graduation), or, as shown, as a tick box element. Any other selection elements for sequential or concurrent selection of ROIs are envisaged herein.

Upon selection by ROI selector 580, the display panel DTP, DVHP is updated so that the now selected regions of interest are displayed in a manner so as to distinguish from other currently non-selected ones. For example, in dose distribution display panel DDP, the selected ROI segmentation may be rendered in different colors or hues as opposed to the non-selective regions of interest that may be rendered in a different color, or in the same colors but in washed-out rendition. In addition or instead, selection may be coded by different line styles that may be used to circumscribe and/or distinguish selected vs non-selected ROIs.

A similar display technique to distinguish selection status may be implemented in the optional dose volume histogram panel DVHP. Dose volume histogram of the currently selected template/selected ROI may be indicated in a heavy line 590 for example, as opposed to dashed lines for histograms 560,570 of (currently) non-selected templates/ROIs. Instead of by dashed vs solid line style selection status may be modulated by line heaviness or different line transparencies or in any other graphical manner. Thus, the display panels DDP, DVHP may themselves be interactive in the output explorer interface UI-O. Other selection/de-selection protocol may be envisaged herein to distinguish different ROI selection states.

Turning now to the embodiment in Fig. 6, this shows an embodiment with graphical user element g(UI-O) of the input explorer user interface UI-I that is configured to for simultaneous selection of multiple templates at once, for example by tick box bar 640 or other. In addition, or instead, a slider bar may be used, similar to the one in Fig. 5.

Upon selection of multiple templates, display panel 630 is adapted to include multiple panels corresponding in number to the templates selected by selector element 640, 650 of the user input data explorer UI-O. In the example of Fig. 6, four panels 630a-d are shown, corresponding to a selection of four templates, for example templates T2, T4, T6 and T9. An optional information box 610 may be displayed with information d(Ti) on the respective templates, similar to information conveyed by widgets 530,540 in Fig. 5.

As will be understood from the above described embodiments, the user interfaces UI-I,UI-O as envisaged herein are so configured to allow either one of two selection options in the input data or the output data as desired. Preferably, there is dynamic update feedback that operates bi-directionally. A change of selection of templates will cause an update of displayed dose distribution/ROIs, whilst a change of selection among ROIs in the display panel is fed back to the input interface side (displayed) information on templates may be updated for example information widget 540. However, a main interest herein is that the UI supports exploring the template vs dose distribution relationship in that different selections of templates are dynamically translated into the displaying of the associated different dose distributions as computed by model M. The user can thus ascertain how the dose distribution changes with the template to so find the right template for the current patient. The dose changes may be shown against a selection of ROIs as backdrop. As another option for yet better dose exploration, a multi-selection of templates in input interface UI-I is translated into a cine-sequence. Thus, a motion picture clip is displayed in panel DDP,DVHP one dose map or histogram after the other, so that the user better appreciates the incurred dose changes. The dose map sequence may be cined against the backdrop of a selection of ROIs as defined by selector 580. The ROI selection may be updated during the cine sequence. A replay may be requested by user if required. For example, the replay may be used to study doses vs different ROI selection backdrops for example. A similar cine sequence may be requested via the GUI for the histograms for a selected number of ROIs. Implementing such dose cines as function of template selections/changes are particularly helpful for the clinical user, as the dose vs template relationship is known to suffer from ill-posedness.

Referring now back to Fig. 2, and turning in more detail to the machine learning model M, this model may be of the generative type. Such models, as opposed to discriminator type models, allow sampling the target space (in this case, the space of dose distributions) by generating instances of dose distributions. Probabilistically speaking, models of the generative type model sampling of the conditional probability distribution $P(y|x)$, with x a template and y dose distribution.

The machine learning model M as used herein may be of the generative type and may be additionally conditioned to produce the respective dose distribution predictions for different template selected as input. This is different from other general generative approaches, such as GAN ("generative adversarial network) that can be trained from random seeds as training input. Such general purpose GANS have been described by Ian Goodfellow et al in "Generative Adversarial Networks", available at arXiv: 1406.2661, 2014. In distinction form this "pure", general GAN approach, it its proposed herein to use as input not random seeds, but respective samples of plan templates to condition the learning accordingly.

However, generative types such as GAN or conditional GANs (cGAN") as described in the literature are not a requirement herein and other models, such as artificial neural networks, are also envisaged herein, trained in for example supervised learning arrangements with training data with input vs target pairs.

In general, in a supervised set up, a machine learning model is pre-loaded with an initial (for example randomly chosen) set of parameters and is then trained on training data including historic templates as training input data, and related dose distributions as targets.

Training instances of training input plan templates and their dose maps as targets may be drawn from existing databases, such as medical databases, medical record databases etc. The contextual data such as the respective planning imagery and/or segmentations may be drawn from image database such as a PACS or others. Instances of plan templates may be generated ad hoc by the user and may be stored for example in a matrix data structure as mentioned earlier in connection with Fig. 4. When generated ad hoc, the user can populate different plan templates matrices with the required information on dose levels priorities etc, and the so populated matrices can then be added to the training database. However, such ad-hoc generated templates are more suitable for unsupervised learning arrangements (also envisaged herein) as the associated dose distribution targets may not be easily come by.

Two operational phases may be distinguished herein in relation to the machine learning model: a learning/training phase and a deployment phase. In training phase, which happens before the deployment phase, the model is trained based on the selected training data using any one or more of a range of different training algorithms as will be described more fully below. Training may be a one-off operation, or may be done repeatedly, for example once new training data becomes available. On completion of the training phase or of one phase thereof if multiple training runs are envisaged, the model may be considered sufficiently trained for deployment and can be used to process new data, for example in day-to-day clinical practice. For example, plan templates may be provided by the user as described above that were not part of the training set to produce the respective predictions of the associated dose distributions when using the explorer system SYS.

A range of different implicit or explicit machine learning model approaches are envisaged herein. Supervised or unsupervised training schemes are envisaged herein. For example, machine learning models envisaged herein include, as mentioned, artificial neural networks, simply referred to as neural networks ("NN"). In particular, regression type networks are envisaged herein such as certain neural network architecture with suitably configured output layers, autoencoders, variational autoencoders, stacked perceptrons, etc.

Whilst neural type networks have been found to be useful herein, this is not to at the exclusion of other types of machine learning model setups. In general, regression type networks are configured to regress plan templates or their indications into respective spatial (image) data that is the dose distributions. As mentioned earlier, machine learning models of the generative type are envisaged herein in particular.

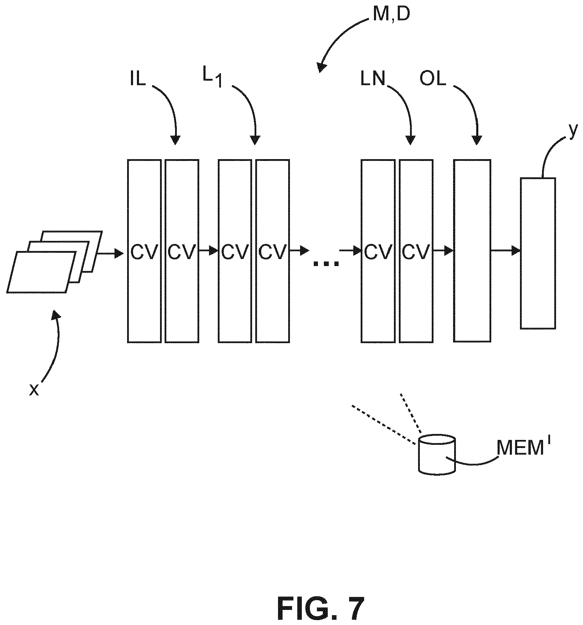

Reference is now made to Fig. 7 which shows machine learning model of the NN type.

The input data including the templates, optionally enriched with contextual data such as planning image, patient records, etc, may be combined into matrices or tensors (higher dimensional matrices). This allows for efficient processing by operators of the various layers Li of the neural network. Preferably, convolutional networks are used including convolutional operators CV. Convolutional networks in particular with a "deep" architecture have been found to yield good results when processing spatially correlated data such as image type data.

Upon input of the input data x, including the plan template data, the model computes/predicts estimates of an associated dose distribution y likely to have arisen if a full planning algorithm had been used, based on the planning template x.

The machine learning model M may be stored in one (or more) computer memories MEM'. The trained model M may be deployed as a machine learning component that may be run on the or a different computing device PU, such as a desktop computer, a workstation, a laptop, etc. or plural such devices in a distributed computing architecture. Preferably, to achieve good throughput, the computing device PU includes one or more processors (CPU) that support parallel computing, such as those of multi-core design. In one embodiment, GPU(s) (graphical processing units) are used.

Generally, the network M may comprise a plurality of computational nodes arranged in the said layers Lj in a cascaded fashion, with data flow proceeding from left to right and thus from layer to layer. As mentioned, convolutional networks have been found to yield good result when processing image type or other spatial data as is the case for the dose maps or planning imagery for example that are processed by the model M.

In deployment, the input data $x$ is applied to input layer IL. The input data x then propagates through a sequence of hidden layers $L_1$-$L_N$ (only two are shown, but there may be merely one or more than two), to then emerge at an output layer OL as a regression result $M(x) = y$, the dose distribution. Thus, the regression output layer OL combines input that it receives from the previous layer $L_N$, and combines this input into the dose distribution $y$.

The model network M may be said to have a deep architecture because it has more than one hidden layer. In a feed-forward network mainly envisaged herein, the "depth" is the number of hidden layers between input layer IL and output layer OL. The number of hidden layers envisaged herein may well run into the 10s or even 100s or more, but a smaller number of hidden layers in single digits are not excluded herein.

Preferably, the hidden layers include a sequence of convolutional layers, represented herein as the said layers $L_1$ - $L_N$. The number of convolutional layers is at least one, such as 2-5, or any other number. The number may run into double-digit figures.

In embodiments, downstream of the sequence of convolutional layers there may be one or more fully connected layers. Alternatively, convolutional and fully connected layers may be interleaved. The output layer OL may be implemented as a full connected layer or as plural fully connected layers.

The hidden convolutional layers $L_j$ and optionally the input layer IL may implement one or more convolutional operators CV. Each layer $L_j$ may implement the same number of convolutional operators CV (or "filters"), or the number may differ for some or all layers. Convolutional operator CV implements a convolutional operation to be performed on its respective input. The convolutional operator may be conceptualized as a convolutional kernel. It may be implemented as a matrix including entries that form filter elements (weights) that form at least a part of the model parameters 0 to be learned. It is in particular these weights that are adjusted in the learning phase.

The first layer IL processes, by way of its one or more convolutional operators, the input data of such input, such as the plan $\tilde{x}$, in particular the plan template, either explicitly or implicitly encoded in suitable image data as will be explained in more detail below. Feature maps are the outputs of convolutional layers, one feature map for each convolutional operator in a layer. The feature map of an earlier layer is then input into the next layer to produce feature maps of a higher generation, and so forth with the last layer OL computing the desired dose distribution result $\tilde{y}$.

Convolutional filters CV in a given layer $L_j$ process an input feature map from an earlier layer into intermediate output, sometimes referred to as logits. An optional bias term may be applied by addition for example. An activation layer $A$ processes in a non-linear manner the logits into the next generation feature map which is then output and passed as input to the next layer, and so forth. The activation layer $A$ may be implemented as a rectified linear unit ("RELU") or as a sigmoid-function, *tanh*-function or any other suitable non-linear function.

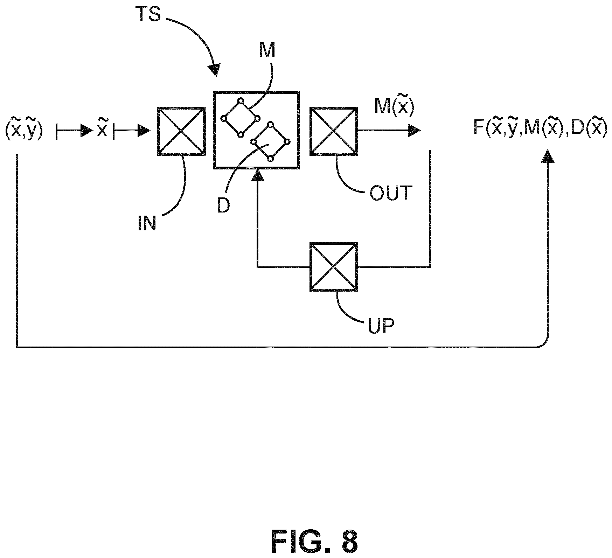

Reference is now made to Fig. 8 which shows a training system TS for training machine learning model M.

As mentioned, a generative setting may be used. In the above-mentioned GAN approach, a type of generative modelling, the predicted model M is embedded in a training framework including a discriminator D and a generator. The model M of interest is trained as the generator part of this framework. Once sufficiently trained, the generator can be made available as the trained machine learning model M. The framework including the discriminator part is then no longer required, but may be retained for future training runs.

Broadly, the training set-up TS includes an updater UP that updates parameters of the model M in light of the training data $\tilde{x}$ as applied to the model. The training data is applied to the model, processed by the current parameterization of the model and output is provide by model as $M(\tilde{x})$. The output will in general differ from the associated target $\tilde{y}$. This deviation is measured by an objective function $F$. The tilde "~" notation indicates that the data is training data, as opposed to new data x which is encountered in deployment to produce predicted dose y.

Optimization schemes such as backward propagation or other gradient based methods are used to adjust the parameter so that the objective function $F$, often configured as a cost function, is improved. In particular, the current parameters 0 are adapted so that the deviation between target $\tilde{y}$ and the training output $M(\tilde{x})$ is reduced.

In GAN setups, a special cost function is used that incorporates conflicting objectives for the two models, the generator M and the discriminator D. In the GAN-learning algorithm, parameters of the models are adjusted in iterations until an equilibrium state is reached, at which point the generator may be deemed trained. Model M is released from the framework as the trained model for use in dose explorer system SYS as described above.

Specifically, a conditioned model M may be used herein, conditioned on different template types. For example, the GAN setup may be implemented as follows. For a given patient, training data $\tilde{x}$ may include planning images, regions of interest, labels/ types of planning templates, planning templates as such (such as described in connection with Fig 4 above) and dose distributions $\tilde{y}$ corresponding to planning templates. During training, the generator M and the discriminator D of the GAN setup are conditioned by the planning template label (e.g., class label or text description). The conditioning is performed by feeding the label into both generator and discriminator as an additional input layer. One trained in a number of iterations using the GAN-cost function, the generator network M is then released from the framework as model M for system SYS. The model M is now trained to predict dose distributions from input data x encountered during deployment, such as from a patient image, regions of interest, and labels/types of planning templates or the plan templates themselves.

In some embodiments, a special GAN cognate may be used, referred to as conditional generative adversary networks cGANs, and described in M Mirza et al in "Conditional Generative Adversarial Nets", available online as arXiv preprint arXiv:1411.1784, (2014), or in G Chrysos et al, "Robust Conditional GAN", published in Int. J. Comput. Vis., vol 128, pp 2665-2683, (2020). However, a general GAN setup is sufficient for present purposes, if the generator is not seeded on random number/data, but instead on purposefully selected, different plan template types.

Instead of using a set of planning templates, it may be beneficial herein to use instead certain surrogates, such as labels or categories of templates, distilled as classes of plan templates from historical sets. This can be done by using an encoder-decoder (ED) network such as autoencoder (AE), optionally of the variational type. EDs or AEs may be implemented in NN architecture with convolutional and deconvolutional operators, to effect dimensional changes of the processed data. Due to the dimensional reduction capability of such networks, caused by the bottleneck architecture, and the setup of its objective function $F$ configured for recovering the input $\tilde{x}$ from the output $\tilde{y}$, a low-dimensional representation (such as a vector, scalar labels, etc) of the input templates in the network's latent space can be learned. The low dimensional representation may then be used as input template type, in lieu of the templates themselves. Encoding templates into classes/labels first, instead of using the templates as such (which is also envisaged herein), may make the learning more robust or efficient, with better performance of the trained model. Instead of autoencoders, other dimensional reduction techniques may be used, such as principal components or clustering techniques such as k-means or nearest neighbors. In an alternative, manual embodiments, the templates may be manually grouped by user through a user interface into classes/types.

In another learning setup (which does not require a generative adversarial network), other image data are used as surrogates for the planning templates. Thus, the plan templates may be represented by an image (e.g., 3D volume) that suitably encodes which OARs may allowed to be exposed to higher radiation ("sacrificed") to the benefit of other OARs that are thus spared at a higher degree. The so sacrificed OARs are thus allowed to absorb a relatively high radiation dose. This sparing-sacrificing distribution may be encoded by a binary label mask for example. The image volumes thus representing the planning templates (or planning intentions) may be used instead or as an additional input channel during training of a neural network M for dose prediction. In this manner, the neural network M can learn the relationship between these additional inputs and the resulting dose distribution. For deployment, these additional input volumes can be created by the user, for example by designating in a planning image, which OARs are to be spared and which are to be sacrificed. Alternatively, a set of different combinations of spared-vs-sacrificed ROIs is established and are then applied as input for the so trained model to predict a variety of possible dose distributions reflecting the various planning intentions/templates. The forming of the various sparing-vs-sacrificing ROI combinations may be facilitated by the input user interface UI-I. A pointer tool may be used to build up various binary masks for example.

In embodiments, an unsupervised training setup may be used to learn differences/types/categories in a set of dose distributions as held in a database of plans. Such a model may be trained with different templates. Therefore, an explicit class label identifying the planning template is not required for training. Instead, latent variables associated with differences in the existing pool of dose distributions are identified during the training process. Thus, the model learns to distinguish different categories of dose distributions that represent the said template types. Latent variables can be categorical or continuous. Multiple sets of categorical and/or continuous variables can be inferred from the training data. The training dataset for such as setup may include a combination of planning images, regions of interests, dose distributions, and vectors of latent variables. The dose distributions can be planned beforehand from different planning templates using planner PS. In deployment, user provides such as model via input interface UI-I with input $\tilde{x}$, including for example, a planning CT image, ROI(s), and one (or more) latent variable(s) $\bar{x}_i$. The output of the model is the dose distribution the model learned to associate with the latent variable $\bar{x}_i$. As an alternative, clustering algorithms may be used to obtain representations of the latent variables.

Unsupervised training approaches for training model M and to learn predicting image type data (such as are dose distributions) and associations between such image data and latent variables may be based on using an information maximizing artificial network, such as descried in Xi Chen et al in "InfoGAN: Interpretable Representation Learning by Information Maximizing Generative Adversarial Nets", published in NIPS 2016, pp 2172-2180.

In general, a training database of plans corresponding to different templates may be generated once for a number of patients by e.g., by manual planning or automated-planning (e.g. auto-planning, multi-criteria optimization, etc.). For example, in a planning template T1, the planner spares organ OAR1 as good as possible while sacrificing organ O2, whereas in planning template T2, the planner spares organ OAR2 as good as possible while sacrificing organ O1, etc.

It should be noted that the templates may be described explicitly as input x, for example, as information such as dose limits in relation or PT, OARs, their priories etc as described above. However, such an explicit provisioning of the templates may not necessarily acquired at all times, as these can also be submitted as input implicitly, for example by suitable surrogate data such as imagery that can be said to represent the respective template. as mentioned above. Alternatively, specifications of certain side effect may be used as surrogate input as these too may be known to be related to different plan templates. Thus, input x may relate to explicitly formulated templates, or may relate implicit templates, formulated in terms of surrogate information known to be correlated to certain template types.

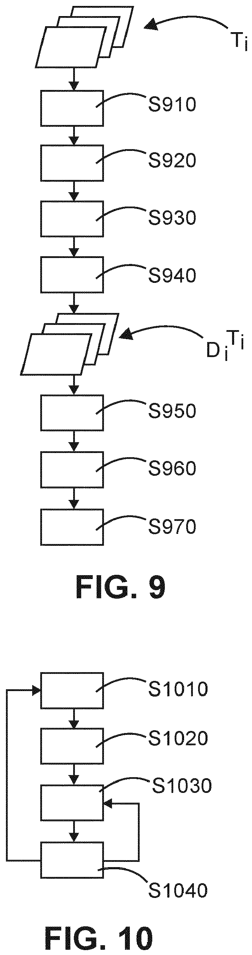

Reference is now made to Figs. 9 and 10 that show respective methods in relation to the above described system SYS. However, it should be understood that the methods are not necessarily tied to the architecture as described above, but may be understood instead as a teaching in their own right.

Turning now to Fig. 9, this shows a flow chart of a computer-implemented method for supporting radiation therapy planning. The method allows identifying a suitable plan template before committing to a full planning with a planning algorithm on the planning station. Consuming unnecessary computational and time resources based on useless plan templates can thus be avoided. First, different templates are explored by the method to compute predictions of associated dose distributions. The predicted dose distribution may be displayed for visual review although other than visual analysis is also envisaged herein. Using a user interface that supports the user in exploring the predicted dose distributions, a suitable such dose distribution may be found. The plan template associated with the so identified dose distribution templates may then then be submitted for planning. The method may be preferably supported by a suitable user interface infrastructure such as graphical user interfaces, to allow user efficiently exploring the input (the plan templates) and/or the output including dose distributions, optionally in relation to ROIs.

Referring first to step S910, plural treatment templates $T_i$ are received in sequence or at once. The treatment plans may be provided by interfacing and retrieval operations controlled by a user interface. The interface allows activating suitable search and interface logic to effect interfacing with for example database(s) of plan templates, and searches in such database(s). The treatment plans are received in a selection operation effectible by user through the user interface. Some or all user interfaces envisaged herein are preferably supported as GUIs, although this is not a requirement herein as textual selection means via keyboard for example are also envisaged.

At step S920 all or some of the selected templates are displayed on a display device in a display operation. Display may be concurrently for all or some plan templates, or in sequence. The plan template includes data that may be used by a planning algorithm to compute a dose distribution and/or delivery device control parameters to achieve the/a dose distribution. The plan template data may describe desirable properties of the dose distributions, for example in terms of upper or lower dose levels, preferably spatially resolved per ROI (eg, PTV or OARs). Additionally, the plan template data may include priority data for the respective dose level targets.

At step S930 a trained machine learning model is used to process the selected plan templates one by one or at once/in parallel, to produce associated dose distributions. The dose distributions are stored in association with their respective plan template as selected in step S930.

The dose distributions are thought to represent predictions of how dose distributions may look like if a planning algorithm (usually an iterative optimization procedure driven by improving an objective function) had been used based on information in the selected templates. Each of dose distributions is associated with its template from which it has been computed by the machine learning model.

At step S940 the predicted dose distributions are displayed, preferably not necessarily in association with their respective plan template. Display of the output may be one by one, at once or in groups as required. Optionally, the ROI(s) may be displayed concurrently with dose distributions maps, for example as an overlay.

At step S950, from among the displayable predicted dose distributions, a suitable one is selected by user or is flagged by an automated quality metric tool. The tool may compute quality metric(s), and identifies the "best" dose distributions relative to the metric. Thus, whilst the predicted dose distributions may be explored manually via the output interface, semi-automatically or fully automatic exploration and analysis is not excluded herein.

At step S960, the plan template associated with the identified best/desired dose distribution may be forwarded to planning system to execute the planning algorithm.

At an optional step S970, the plan is computed. Computation of the plan may include mapping to control parameters/fluence maps to obtain data/parameter that allow controlling the treatment delivery device TD to deliver the dose to the patient during treatment.

The plan template selection step S910 and the dose distribution display step S940 are preferably coupled in a dynamic feedback loop: the display step is updated as the new templates or combinations thereof are selected at step S910.

The display step S940 may include a cine operation to display a motion picture clip of the predicted dose distributions associated with the current set of selected plan templates. The motion picture clip may be looped repeatedly (replay) and/or may be updated in response to new or de-selection(s) at step S910.

Referring now to the computer implemented method in Fig. 10 this shows a training method for training a machine learning model.

The steps may include finding training data and applying the same to a learning algorithm to adapt a model. The model may be implicit or explicit. Implicit modeling includes clustering or other approaches where essentially the training data set itself represents the model, as opposed to explicit modelling where a parametrized function (or system of such functions) is used in the modelling, logically distinct from the training data itself. NN type modelling is an example of explicit modeling. This distinction however is not a dichotomy however, as hybrid modelling, combining aspects from both approaches, are also envisaged herein.

The following steps may describe in more detail a supervised learning scheme based on training data. The training data includes training input data and associated targets. For example, training input x may relate to templates or surrogates thereof, and $y$ may relate to the respective associated dose distributions. The training data may include plural such instances i of input vs, target pairs $(\tilde{x}, \tilde{y})^i$. The training data may be synthetically generated ad-hoc by the user, or may be sourced through search and retrieve from historical data from previous RT sessions for same or for different patients, as may be held in medical databases. A scripted tool may be used to automate identification, retrieval and collation of the training data.

Broadly, the training method includes receiving training data. Based on the training data, parameters of model M are adapted. The adaption may be iterative, and controlled by objective function $F$ such as cost function $F$. Cost function $F$ is configured to measure how well the model performs.

In more detail, and in some embodiments, at step S1010 training data is received in the form of pairs $(\tilde{x}_k, \tilde{y}_k)$. Each pair includes the training input $\tilde{z}_k$ and the associated target $\tilde{y}_k$. $\tilde{x}_k$, as defined above.

At step S1020, the training input $\tilde{x}_k$ or plural such training inputs of a batch is applied to an initialized machine learning model M to produce a training output.

A deviation, or residue, of the training output $M^\theta(\tilde{x}_k)$ from the associated target $\tilde{y}_k$ is quantified by a cost function $F$ at step S1030. One or more parameters of the model are adapted at step S1040 in one or more iterations in an inner loop to improve the cost function. For instance, the model parameters are adapted to decrease residues as measured by the cost function. The parameters include in particular weights of the convolutional operators, in case a convolutional NN model M is used.

The training method then returns in an outer loop to step S1010 where the next pair of training data or a next batch of such training data pairs is fed in. In step S1020, the parameters of the model are adapted so that the aggregated residues of all pairs considered are decreased, in particular minimized. The cost function quantifies the aggregated residues. Forward/backward propagation or similar gradient-based techniques may be used in the inner loop.

Cost function $F$ measures the aggregated residue(s), that is, the error incurred between data estimated by the model M and the targets as per some or all of the training data pairs $k$:

$$argmin_\theta F = \sum_k || M^\theta(\tilde{x}_k) - \tilde{y}_k || \qquad (1)$$

In eq. (2) and below, function $M()$ denotes the result of the model M applied to training input $\tilde{x}$.

In training, the training input data $\tilde{x}_k$ of a training pair is propagated through the initialized network M. Specifically, the training input $\tilde{x}_k$ for a $k$-th pair is received at an input IL, passed through the model and is then output at output OL as output training data $M^\theta(\tilde{x}_k)$. A suitable similarity measure $|| \cdot ||$ is used to measure the deviation, also referred to herein as residue, between the actual training output $M^\theta(\tilde{x}_k)$ produced by the model M, and the desired target $\tilde{y}_k$. For regression learning, least squares or more general $l_p$ measures may be used. In GAN setups, the cost function is formulated in terms of cross (binary) entropy loss in minmax setup, with a further dependency in the cost function F on the output of discriminator $D(\tilde{x})$ component of the GAN learning framework. In clustering type modelling approaches, the cost function is formulated in terms of metrics of partitions of the training data set.

The components of the SYS may be implemented as one or more software modules, run on one or more general-purpose processing units PU such as a workstation associated with the delivery device TD, or on a server computer associated with a group of imagers.

Alternatively, some or all components of the SYS may be arranged in hardware such as a suitably programmed microcontroller or microprocessor, such an FPGA (field-programmable-gate-array) or as a hardwired IC chip, an application specific integrated circuitry (ASIC), integrated into the imaging system SYS. In a further embodiment still, the system SYS may be implemented in both, partly in software and partly in hardware.

The different components of the system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

**Claims**

1. A computing system (SYS) for supporting radiation therapy planning, comprising:

   an input interface (IF1; UI-I) for receiving input radiation treatment plan templates, or types of such plan templates, for plural radiation treatment plans, and
   a trained machine learning model (M) configured to compute plural dose maps associated with the received radiation treatment plan templates or with the types of such plan templates.

2. System of claim 1, wherein the input interface (UI-I) is arranged as a user interface, UI, configured to allow a user to select the input radiation treatment plan template or type.

3. System of claim 2, wherein the input interface (UI) is configured to allow selection of any one or more of the input templates and the plural dose maps are computed by the trained machine learning model in response to such selection.

4. System of claim 1 or 2, further comprising an output navigation user interface (UI-O), configured to allow a user to navigate through the output plural dose maps and select a dose map from among the plural dose maps.

5. System of claim 3, wherein the output navigation user interface (UI-O) and/or the input interface (IF1) is arranged as a graphical user interface, GUI.

6. System of any one of the previous claims, wherein the trained machine learning model is of the generative type.

7. System of any one of the previous claims, wherein the templates include data that relates to different clinical goals.

8. System of claim 6, wherein the said clinical goals includes any one or more of: i) different clinical dose upper limits for one or more organs at risks, ii) different dose lower limits for a target, iii) different trade-offs between target coverage and organ-at-risk sparing iv) different treatment parameters

9. System of any one of the previous claims, including a radiation therapy planning module (PM) configured to generate a treatment plan based on the dose map selected by the user via the output navigation interface (UI-O).

10. A training system (TS) configured to train a machine learning model (M) based on training data to obtain the trained model of the system of any one of the previous claims.

11. A radiation treatment arrangement, including a planning system (PS), a system of any one of previous claims, and/or a treatment delivery device (TD).

12. A computer-implemented method, comprising:

    receiving (S910) input radiation treatment plan templates, or types of such plan templates, for plural radiation treatment plans, and
    computing (S930) by a trained machine learning model (M) plural dose maps associated with the received radiation treatment plan templates or with the types of such plan templates.

13. Method for training a machine learning model (M) based on training data to obtain the trained model in the system of any one of the previous claims.

14. A computer program element, which, when being executed by at least one processing unit (PU), is adapted to cause the at least one processing unit (PU) to perform the method as per any one of claims 12 or 13.

15. At least one computer readable medium having stored thereon the program element of claim 14, or having stored thereon the trained machine learning model (M) as per any one of previous claims.

**FIG. 1**

FIG. 2

# FIG. 3

**FIG. 4**

**FIG. 5**

FIG. 6

**FIG. 7**

**FIG. 8**

$T_i$

S910

S920

S930

S940

$D_i{}^{T_i}$

S950

S960

S970

**FIG. 9**

S1010

S1020

S1030

S1040

**FIG. 10**

Europäisches Patentamt

European Patent Office

Office européen des brevets

# EUROPEAN SEARCH REPORT

**Application Number**

EP 21 20 7247

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/333623 A1 (HIBBARD LYNDON STANLEY [US]) 31 October 2019 (2019-10-31)<br>* paragraph [0001]; figures 1,3,4 *<br>* paragraph [0004] *<br>* paragraph [0025] *<br>* paragraph [0026] *<br>* paragraph [0027] *<br>* paragraph [0030] *<br>* paragraph [0034] *<br>* paragraph [0041] *<br>* paragraph [0044] *<br>* paragraph [0051] *<br>* paragraph [0055] *<br>* paragraph [0057] *<br>* paragraph [0081] *<br>* paragraph [0083] *<br>* paragraph [0090] *<br>* paragraph [0096] – paragraph [0097] *<br>* paragraph [0101] *<br>* paragraph [0104] *<br>* paragraph [0114] *<br>───── | 1-15 | INV.<br>A61N5/10<br>G16H20/40<br>G06N3/08<br>G06N20/00<br>G16B40/00 |
| X | US 2018/178036 A1 (LAAKSONEN HANNU [FI] ET AL) 28 June 2018 (2018-06-28)<br>* paragraph [0012] *<br>* paragraph [0039] – paragraph [0041] *<br>* paragraph [0049] *<br>* paragraph [0052] *<br>* paragraph [0054] *<br>* paragraph [0057] – paragraph [0058] *<br>* paragraph [0041] *<br>───── | 1-5,7-15 | TECHNICAL FIELDS SEARCHED (IPC)<br>A61N<br>G16H<br>G06N<br>G06F<br>G16B |
| A | US 2013/197878 A1 (FIEGE JASON [CA] ET AL) 1 August 2013 (2013-08-01)<br>* paragraph [0182] – paragraph [0183] *<br>───── | 1-15 | |

−/−−

The present search report has been drawn up for all claims

| Place of search | Date of completion of the search | Examiner |
|---|---|---|
| Munich | 1 April 2022 | Rodríguez Cosío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Europäisches Patentamt
European Patent Office
Office européen des brevets

**Application Number**

EP 21 20 7247

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2017/173365 A1 (BZDUSEK KARL ANTONIN [US] ET AL) 22 June 2017 (2017-06-22) <br> * paragraph [0014] * <br> * paragraph [0025] * <br> * paragraph [0033]; claim 8 * <br> * paragraph [0035] – paragraph [0036] * <br> * paragraph [0038] * | 1-15 | |
| A | EP 3 744 395 A1 (VARIAN MEDICAL SYSTEMS INT AG [CH]) 2 December 2020 (2020-12-02) <br> * paragraph [0045] * <br> * paragraph [0029] * <br> * paragraph [0043] * <br> * paragraph [0046] * <br> * paragraph [0085] * | 1-15 | |
| Y | US 2018/211725 A1 (PURDIE THOMAS G [CA] ET AL) 26 July 2018 (2018-07-26) <br> * paragraph [0021] * <br> * paragraph [0022] * <br> * paragraph [0155] * <br> * paragraph [0185] – paragraph [0186] * <br> * paragraph [0188] * <br> * paragraph [0206] – paragraph [0207] * | 1-15 | TECHNICAL FIELDS SEARCHED (IPC) |
| Y | US 2019/076671 A1 (WILLCUT VIRGIL MATTHEW [US] ET AL) 14 March 2019 (2019-03-14) <br> * paragraph [0040] * <br> * paragraph [0045] * <br> * paragraph [0099] * | 1-15 | |

The present search report has been drawn up for all claims

| Place of search | Date of completion of the search | Examiner |
|---|---|---|
| Munich | 1 April 2022 | Rodríguez Cosío, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 176 926 A1

## ANNEX TO THE EUROPEAN SEARCH REPORT
## ON EUROPEAN PATENT APPLICATION NO.

EP 21 20 7247

This annex lists the patent family members relating to the patent documents cited in the above-mentioned European search report.
The members are as contained in the European Patent Office EDP file on
The European Patent Office is in no way liable for these particulars which are merely given for the purpose of information.

01-04-2022

| Patent document cited in search report | | Publication date | Patent family member(s) | | Publication date |
|---|---|---|---|---|---|
| US 2019333623 | A1 | 31-10-2019 | AU | 2019262835 A1 | 17-12-2020 |
| | | | CN | 112041026 A | 04-12-2020 |
| | | | EP | 3787744 A1 | 10-03-2021 |
| | | | JP | 2021522886 A | 02-09-2021 |
| | | | US | 2019333623 A1 | 31-10-2019 |
| | | | WO | 2019212804 A1 | 07-11-2019 |
| US 2018178036 | A1 | 28-06-2018 | EP | 3342461 A1 | 04-07-2018 |
| | | | US | 2018178036 A1 | 28-06-2018 |
| US 2013197878 | A1 | 01-08-2013 | CA | 2796159 A1 | 15-12-2011 |
| | | | EP | 2575965 A2 | 10-04-2013 |
| | | | US | 2013197878 A1 | 01-08-2013 |
| | | | WO | 2011153639 A2 | 15-12-2011 |
| US 2017173365 | A1 | 22-06-2017 | CN | 105793854 A | 20-07-2016 |
| | | | EP | 3077936 A1 | 12-10-2016 |
| | | | JP | 6602299 B2 | 06-11-2019 |
| | | | JP | 2016538944 A | 15-12-2016 |
| | | | US | 2017173365 A1 | 22-06-2017 |
| | | | WO | 2015083035 A1 | 11-06-2015 |
| EP 3744395 | A1 | 02-12-2020 | CN | 110114117 A | 09-08-2019 |
| | | | EP | 3562549 A1 | 06-11-2019 |
| | | | EP | 3741431 A1 | 25-11-2020 |
| | | | EP | 3744395 A1 | 02-12-2020 |
| | | | US | 2018185669 A1 | 05-07-2018 |
| | | | US | 2020009401 A1 | 09-01-2020 |
| | | | WO | 2018122251 A1 | 05-07-2018 |
| US 2018211725 | A1 | 26-07-2018 | CA | 2951048 A1 | 18-12-2014 |
| | | | EP | 3007771 A1 | 20-04-2016 |
| | | | US | 2016140300 A1 | 19-05-2016 |
| | | | US | 2018211725 A1 | 26-07-2018 |
| | | | WO | 2014197994 A1 | 18-12-2014 |
| US 2019076671 | A1 | 14-03-2019 | AU | 2018331365 A1 | 30-04-2020 |
| | | | CN | 111417435 A | 14-07-2020 |
| | | | EP | 3681600 A1 | 22-07-2020 |
| | | | JP | 6896164 B2 | 30-06-2021 |
| | | | JP | 2020533123 A | 19-11-2020 |
| | | | US | 2019076671 A1 | 14-03-2019 |
| | | | WO | 2019055491 A1 | 21-03-2019 |

EPO FORM P0459

For more details about this annex : see Official Journal of the European Patent Office, No. 12/82

29

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL.** Machine Learning. McGraw-Hill, 1997, 2, , 6
- **B. E. NELMS et al.** Variation in external beam treatment plan quality: An inter-institutional study of planners and planning systems. *Pract. Radiat. Oncol.,* 2012, vol. 2 (4), 296-305
- **IAN GOODFELLOW et al.** Generative Adversarial Networks. *arXiv: 1406.2661,* 2014
- **M MIRZA et al.** Conditional Generative Adversarial Nets. *arXiv:1411.1784,* 2014
- **G CHRYSOS et al.** Robust Conditional GAN. *Int. J. Comput. Vis.,* 2020, vol. 128, 2665-2683
- **XI CHEN et al.** InfoGAN: Interpretable Representation Learning by Information Maximizing Generative Adversarial Nets. *NIPS,* 2016, 2172-2180